# EUROPEAN PATENT APPLICATION

(11) **EP 4 413 970 A1**
(43) Date of publication of application: **14.08.2024**
(21) Application number: 23162913.0
(22) Date of filing: 20.03.2023
(51) Int. Cl.: A61K 8/19, A61K 8/22, A61K 8/34, A61K 8/38, A61K 8/41, A61K 8/92, A61Q 11/00

(54) **COMPOSITION AND METHOD FOR TOOTH WHITENING WITH SENSITIVITY RELIEF**

(30) Priority: 08.02.2023 US 202363444088 P
(71) Applicant: Koninklijke Philips N.V., 5656 AG Eindhoven (NL)
(72) Inventor: MAO, Jingliang, 5656 AG Eindhoven (NL); JACINTO, Sergio, 5656AG Eindhoven (NL)
(74) Representative: Philips Intellectual Property & Standards

(57) **Abstract**

Tooth whitening compositions, such as used in tooth whitening pens, and methods related thereto. The tooth whitening composition includes a peroxide component, a carrier for the peroxide component, a pH modifying component, and a tooth desensitizing agent that is compatible with the peroxide component. The composition is a homogenous mixture that is substantially anhydrous, that has a viscosity exceeding about 1000 cP, that has a pH ranging between about 6.5 and about 7.0, and that provides tooth sensitivity relief upon application.

## Description

### FIELD OF THE INVENTION

The present disclosure relates generally to tooth whitening compositions such as used in tooth whitening pens, and methods related thereto. The composition includes a peroxide component in a homogenous and viscous mixture of near neutral pH that also provides sensitivity relief to a user.

### BACKGROUND

Tooth whitening has become a popular cosmetic activity. Tooth whitening can refer to either the restoration of a natural tooth shade or the whitening of a tooth beyond the natural shade. Typically, tooth whitening is accomplished by bleaching with hydrogen peroxide (HP) or carbamide peroxide (CP), or a mixture of both. Options for techniques to accomplish tooth whitening include the use of whitening pens, toothpastes, mouth rinses, chewing gums, paint-on films, in-office bleaching methods, and the application of whitening gels with trays or plastic strips, either over-the-counter or from a dentist. Among them, the use of a whitening pen is the most simple, direct, and convenient way.

Typically, to maintain good retention of the active ingredient on teeth surfaces during a whitening treatment and to prevent saliva from washing away the active ingredient, applicators, such as tooth whitening pens, require use of highly viscous solutions, emulsions, or gels, or the formation of films of either HP or a mixture of HP and CP.

For example, U.S. Pat. No. 2007/0189983A1 describes an anhydrous liquid tooth whitening composition including an orally acceptable carrier having a bio-adhesive agent and a film-forming agent. As another example, US. Pat. No. 8,137,658 describes a tooth whitener composition for delivery of active H₂O₂ in a film-forming complex to provide bio-adhesion and retention of the composition on teeth.

Currently, most tooth whitening pen products use either HP alone or a mixture of HP- and CP-based compounds in a viscous hydrophilic medium. Regardless of differences in dosing devices, compositions used in existing tooth whitening pens may have one or more of the problems or disadvantages described below.

Existing compositions may have safety issues when specific chemical compounds are added to the whitening product to boost the stability of HP. Some of the stabilizer compounds may be unsafe for users.

Existing compositions may carry a risk to cause demineralization of the tooth enamel when the pH of a product falls below 6.5. In an aqueous environment, to keep hydrogen peroxide stable at room temperature for a long period of time, either an acidic pH, *i.e.,* below 4.5, is needed or an effective HP stabilizing agent needs to be present, or both. The pH of most existing whitening pen products is typically below pH 6.5 to keep HP stable. However, the "critical pH" for tooth enamel demineralization is pH 5.5, yet the "critical pH" for dentin is only pH 6.5. Therefore, when dentin is exposed, it can be eroded much faster than enamel as it is vulnerable to more erosive sources. (See for example *http:*//*colgate.dentalaegis.com*/*pdfs*/*Colgate_WebArticles_Guignon_6th.pdf*). Thus, when the pH of a whitening product drops below pH 6.5, the whitening product poses a demineralization risk to teeth dentine and in some cases, even to teeth enamel.

The existing products may have a short shelf life. As mentioned above, the long-term stability of HP in an aqueous environment is highly affected by the pH of the solution. A pH of 4.5 or lower is desired for HP stability when a stabilizer is not present. However, the consideration of tooth demineralization risk requires that the pH of formulated whitening compounds be above 5.5. Thus, at room temperature or at refrigerator storage temperature, the shelf life of most existing whitening pen products with pH above 5.5 is around one year or less, which is often shorter than the shelf life of other types of whitening products.

The existing products may cause tooth sensitivity that arises during the whitening process. Existing tooth whitening pens lack desensitizing agents, and thus cannot address tooth sensitivity issues and potential discomfort caused by peroxide-based whitening for some users.

### SUMMARY

While existing tooth whitening compositions may function for their intended purpose, there is a continuing need to improve the quality, safety, and efficacy of teeth whitening products, in particular for tooth whitening applications accomplished with a pen-style applicator. According to the implementations and embodiments described herein addressing such a need, compositions and methods for tooth whitening are disclosed.

The disclosure is directed to a composition for tooth whitening, including a peroxide component, a carrier for the peroxide component, a pH modifying component, and a tooth desensitizing agent that is compatible with the peroxide component. The composition a homogenous mixture that is substantially anhydrous, that has a viscosity exceeding about 1000 cP, that has a pH ranging between about 6.5 and about 7.0, and that provides tooth sensitivity relief upon application.

In some aspects, the techniques described herein relate to a composition, wherein a hydrophobic content of the homogenous mixture is more than 50% (w/w). In some embodiments, the peroxide component is selected from hydrogen peroxide, peracetic acid, and other suitable peroxide that is stable in the composition. In some embodiments, the peroxide component is hydrogen peroxide at a concentration of less than about 30% (w/w), for example a composition wherein the concentration of hydrogen peroxide is in a range of about 2.0% (w/w) to about 14% (w/w). In some embodiments, the carrier for the peroxide component is ethanol.

In some embodiments, the composition further includes a thickening agent, for example selected from methacrylic acid copolymer, ammonia methacrylate copolymer, and polyvinyl acetate.

In some embodiments, the carrier for the peroxide component is a non-alcohol, non-toxic, hydrophobic carrier, such as a carrier selected from petrolatum, polyethylene glycol (PEG), and dimethyl silicone fluids.

In some embodiments, the carrier for the peroxide component is a mixture of an alcohol carrier and a non-alcohol, non-toxic, hydrophobic carrier with a compatible thickening agent. In some embodiments, the tooth desensitizing agent is an inorganic salt, for example a potassium salt. In some embodiments, the tooth desensitizing agent is an organic compound, such as an organic compound selected from eugenol, menthol, and other organic tooth sensitivity relief agent. In some embodiments, the pH modifying component is selected from potassium hydroxide, sodium hydroxide, and other basic chemicals.

In some aspects, the techniques described herein relate to a tooth whitening kit, including a composition for tooth whitening as disclosed herein and a dispensing device configured to receive the composition as a single part and adapted to dispense the composition onto a user's teeth.

In some aspects, the techniques described herein relate to a method for whitening teeth, including providing a composition for tooth whitening as disclosed herein and applying the composition to a tooth of a user.

In some aspects, the techniques described herein relate to a method of manufacturing a composition for tooth whitening, including the steps of providing a peroxide component, a carrier for the peroxide component, a pH modifying component, and a tooth desensitizing agent that is compatible with the peroxide component; and combining the peroxide component, the carrier, the pH modifying component, the tooth desensitizing agent into a homogenous mixture that is substantially anhydrous, that has a viscosity exceeding about 1000 cP, that has a pH ranging between about 6.5 and 7.0, and that provides tooth sensitivity relief upon application.

By using compositions and methods of the current disclosure, the inventive subject matter will not only overcome problems described above, but also provide significant improvements to the safety of use, reduce the risk of demineralization, improve the shelf life and stability of the composition, and provide sensitivity relief of tooth whitening products applied with a pen-type applicator.

### BRIEF DESCRIPTION OF THE DRAWINGS

FIG. 1 is perspective view of an exemplary tooth whitening kit using a pen to hold and apply a tooth whitening composition.
FIG. 2 is a schematic illustration of the application of a tooth whitening composition as disclosed herein with a tooth whitening pen to a dental surface.
FIG. 3 is a high-level flow chart of an exemplary method of manufacturing a composition for tooth whitening.

### DETAILED DESCRIPTION OF EMBODIMENTS

The inventive subject matter is directed to compositions for tooth whitening including a peroxide component. In some embodiments, the peroxide component is HP.

It is known that water acts as a contaminant in HP solutions. The higher the water concentration, the less stable the peroxide solution is. Hydrogen peroxide becomes more stable when there is less water present in the solution. On the other hand, it is known that pH plays an important role in the stability of HP in an aqueous environment. In general, most commercial aqueous hydrogen peroxide solutions, for example an aqueous solution with less than 50% (w/w) hydrogen peroxide, have a pH within a pH range of about 1.5 to about 4.5. It is well known that hydrogen peroxide solutions are most stable within this range at room temperature.

Water generally exists as bound water and/or free water in a medium. Bound water can be defined as the water molecules held in a medium by molecular or electro-molecular forces. Bound water has more structural bonding than liquid or free water; thus, it is unable to act as a solvent for dissociation of molecules or ionic compounds such as salts, or complexes into other sub-species such as atoms, ions, or radicals. On the other hand, free water molecules can be extracted easily from a medium since the molecules of water are free to move within the limits of the container, as limited by gravity. The available free water molecules have a big impact on the dissociation of hydrogen peroxide to perhydroxyl ions, and thus the decomposition of HP is present when hydroxyl anions are present in an alkaline condition. Hydrogen peroxide is unstable under alkaline conditions. The rate of decomposition increases with rise in temperature, concentration, and pH. The best stability of hydrogen peroxide can be attained with cool, dilute, and acidic conditions. When the pH of the solution is close to pH 7.0, little or no free water in a peroxide compound is very beneficial for the long-term stability of hydrogen peroxide.

Compositions according to the disclosure are created by a combination of both chemical and mechanical means, as further discussed below.

In some embodiments, the composition can be an anhydrous, viscous mixture of near neutral pH made as a single part. As used herein, the term "single part" means the tooth whitening composition is embodied as a single chemical component or is present in a single compartment. The single part contains all the chemicals of a tooth whitening composition needed for tooth whitening. Advantageously, the composition is prepared in the form of a "single part" composition, *i.e.,* all components of the whitening composition are self-contained in a desired pre-mixed proportion. Accordingly, there is no requirement for the components of the composition to be physically separated from each other prior to use in order to avoid any undesirable interactions, as may occur with some existing peroxide-containing formulations.

The tooth whitening compositions disclosed herein are viscous liquids, such as gels, which maintain their consistency during storage, thereby enabling the product to be applied to the tooth surface with a pen-type applicator. The viscosity of the tooth whitening composition disclosed herein may be greater than or equal about 1,000 centipoise (cP) and less than about 300,000 cP. The mixture can be a homogeneous mixture characterized by a uniform dispersion of its constituent substances throughout. The mixture can be in the form of solutions, suspensions, emulsions, or colloids.

As used herein, the term "anhydrous" means that no free water is used in the intended product formula and formulation. In other words, there is no direct addition of water to the disclosed compositions. Little or no free water exists in the formulated peroxide compound in the finished product. However, those skilled in the art will recognize that water may be physically and/or chemically absorbed by a composition and/or by one or more ingredients in a composition at any time during the preparation, storage, and/or use of the disclosed compositions (i.e., by indirect addition of water to the composition). The tooth whitening compositions may include a small percentage of water from commercial ingredients used in product manufacture. However, such a small percentage of water does not interfere with the general hydrophobic character of the tooth whitening composition. Since two raw materials such as hydrogen peroxide and alkaline pH adjusting agents in the formulation process cannot be used with 100% purity itself, a small percentage of water is brought into the formulation by those two ingredients' commercial grade products. For example, in production of some tooth whitening products, commercial 50% Cosmetic Grade (CG) HP raw material contains about 50% of HP and about 50% of bound water in its contents.

As used herein, the term "near neutral pH" refers to a pH in the range of about 6.5 to about 7.0.

The chemical components of the tooth whitening compositions disclosed herein may include one or more peroxide components, organic solvents, and anhydrously compatible thickeners, desensitizers, pH modifying agent, flavors, surfactants, and/or other functional agents, depending on the functions desired for the product. The chemicals are selected based on compatibility requirements with the peroxide component. Furthermore, it is understood that in applications for use in the oral cavity, the chemical components and concentration are to be selected based on limits of safety for use in the oral cavity. The disclosed composition provides the benefit of having an extended shelf life. For example, in some embodiments, a composition may have a shelf life of about 18 months.

In production, due to the anhydrous and viscous properties of some ingredients and the finished compound product, special mechanical mixing means are required for compounding in order to produce a viscous and homogenous mixture. For example, those skilled in the art will recognize that, in some embodiments, when the viscosity of a blended compound exceeds 1000 cP and the hydrophobic content is more than 50% of the composition, a colloid mill may be used. Additionally, in some embodiments, vacuum degassing during or after colloid mill processing may be applied. In other embodiments, when gentle blending is required and/or when shear-sensitive ingredients are present, devices such as a double planetary mixer can be used for mixing and kneading viscous compounds. In some embodiments, a strong blender, like a Ross turbo-emulsifier, can be used for the mixing of viscous materials and for homogenizing, emulsifying, dispersion, and particle size reduction.

Depending on specific configuration and performance requirements, embodiments of a single part composition may vary accordingly in this disclosure. Detailed descriptions of example embodiments of compositions and manufacturing methods are provided in sections A through C below.

### A. Alcohol Based, Anhydrous, Near Neutral pH, Peroxide Composition with Sensitivity Relief

### Peroxide Component

As mentioned before, one suitable peroxide component is HP. Concentrations of HP as raw material can be 30%, 35%, 50% or 75% HP and meet the quality requirements set forth by Cosmetic Grade (CG), Reagent Grade (RG), American Chemical Society (ACS) Grade, National Formulatory (NF) Grade, Food Chemical Codex (FCC) or US Pharmacopeia (USP) Grade. Although those concentrations of HP ingredients contain water, during formulation of the composition, those water molecules act as bound water. Water molecules in the commercial HP raw material and in the formulated products form strong hydrogen bonds with hydrogen peroxide molecules, alcohol, and/or other organic molecules containing accessible hydroxyl groups, and thus can be considered as "bound water" but not "free water". The lack of free water keeps HP stable in the finished compound during long term storage. In some embodiments, the concentration of hydrogen peroxide in the tooth whitening composition can be as high as 30% (w/w). Some embodiments of the tooth whitening composition may have a concentration of hydrogen peroxide around about 2.0% to about 14.0% (wt.%).

To make the composition safe for tooth enamel, a suitable pH of the finished composition of the whitening pen can be around pH 6.5 - 7.0. In general, a pH of the composition below 7.0 is advantageous to avoid HP degradation facilitated by hydroxyl ions. The selection of the peroxide component in conjunction with the low water carrier provides stabilized delivery of the whitening agent. The whitening activity is maintained for application to the tooth or oral surface and is maintained through storage.

In other embodiments, it is envisioned that other peroxide compounds can be used, provided that they are stable in the final compositions. In some embodiments, peracids, such as peracetic acid, can be used as a suitable peroxide component. In further embodiments, a peroxide solution can be used. In other embodiments, a suspension of solid peroxide compounds such as carbamide peroxide can be used.

### Thickening Agents

There are many polymers and anhydrous medium components that are compatible with peroxides over a long term and that are generally considered safe for its intended use in oral health care applications. Examples of suitable thickening agents include USP grade of methacrylic acid copolymer, ammonia methacrylate copolymer, and polyvinyl acetate.

### Alcohol Carrier

In this formulation, one of the carriers for the peroxide component is an alcohol. The term "alcohol" refers to a primary alcohol, such as ethanol (ethyl alcohol). In some embodiments, FCC, USP, ACS, RG, and/or NF grade of ethanol without denaturation can be used.

### Tooth Desensitizing Agent

With an appropriate formulation strategy, in some embodiments, peroxide compatible functional desensitizing agents can be used. Examples of peroxide compatible inorganic desensitizing salts include potassium salts such as potassium nitrate (KNO₃).

### pH Modifying Component

Typically, commercial aqueous hydrogen peroxide raw materials have a pH within a pH range of about 1.5 to about 4.5. To attain the desired pH range of about 6.5-7.0 in the final single part composition, pH modifying agents may be added. In some embodiments, alkaline pH modifying agents can be attained using alkali earth or metal or ammonium hydroxides. In some embodiments, pH modifying components can include potassium hydroxides or sodium hydroxides. In one example embodiment, the dosage of an alkaline agent can be about 0.05% to about 0.5% of potassium hydroxide (caustic potash) in the final tooth whitening composition. In other embodiments, other alkaline (basic) salts can be used. In other embodiments, carbonates and/or bicarbonates can be present. In further embodiments, borates, silicates, phosphates, and/or other buffers may be used.

### Flavors

Flavoring agents are substances that trigger the sense of taste and/or smell of the user. They are used to improve the consumer perception and experience in tooth whitening by either adding flavor to an otherwise flavorless product or to mask any undesirable flavors. Generally, for dental products minty flavors are used to give a sense of cleanliness and freshness. Sweeteners are also used but not to the same extent as other consumer products as sweetness is perceived to be detrimental to dental health. In some embodiments, a flavoring agent like natural peppermint oil may be added, partly due to its ready solubility in anhydrous medium.

### Organic Desensitizing Agent

In some embodiments, organic desensitizers, solvents, surfactants, and/or other chemicals may be added to provide a desired function. For example, in some embodiments, organic desensitizers such as eugenol and menthol, which is contained in pepper mint oil, may be added as additional desensitizer or to further enhance the desensitizing effect of inorganic desensitizer. In some embodiments, other suitable organic tooth sensitivity relief agents may be added. In other embodiments, solvents such as petrolatum, propylene glycol, glycerol, isoparaffinic hydrocarbon, and PEG may be added. In further embodiments, lipophilic surfactants like sorbitan monopalmitate can be added.

### Dispensing Device

Due to the single part composition, a dosing and dispensing device for the tooth whitening composition can be a pen-type applicator, single barrel syringe, bottle, or other dispensing device that is capable of dispensing highly viscous fluids. In some embodiments, the tooth whitening composition can be housed in a one-compartment reservoir. The one-compartment reservoir can be a detachable container or cartridge, or it can be a single chamber integrated in the dispensing device.

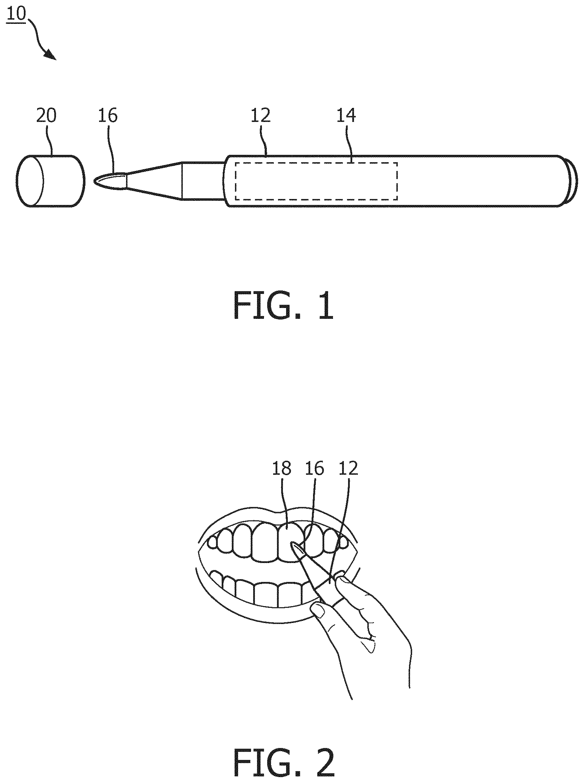

FIGs. 1-2 illustrate an embodiment of a tooth whitening kit 10 including a dispensing device 12 in the form of a pen for holding a tooth whitening composition therein as a single part. The disclosed compositions can be housed in a dispensing tube 14. Dispensing device 12 has as a tip 16, for example a soft felt tip, adapted to dispense the composition onto a user's teeth 18. In other embodiments, the applicator tip can be a brush, sponge, swab or roller.

As illustrated in FIG. 2, dispensing device 12 is configured for being held by a user when applying the tooth whitening composition to a tooth surface. Tip 16 is brought into contact with a tooth surface such that a predetermined amount of the tooth whitening composition is deposited to the tooth. Optionally, dispensing device 12 may be provided with a cap 20 for protection of tip 16 and for allowing easy repeated access.

In some embodiments, the composition may be supplied in a removable cartridge that can be replaced or refilled. In further embodiments, the dispensing device can include a pump or vacuum means to withdraw the composition from the compartment and discharge the composition to the discharge tip. In some embodiments, the dispensing device may dispense the tooth whitening composition through a transfer channel via capillary action, such as in a flow through pen, or through an activator, such as mechanical piston with a click mechanism, twist button and ratchet mechanism, or push button mechanism, or through other such mechanical means for transferring the composition from the dispensing device to a dental surface.

An example formulation of a tooth whitening composition in the form of an alcohol-based, anhydrous, near neutral pH, 4.5% (wt/wt.) hydrogen peroxide compound with sensitivity relief for teeth whitening is set forth in Table 1.

**TABLE 1**

| | **Ingredient** | **% w/w in part (a)** |
|---|---|---|
| Phase I | Hydrogen Peroxide, 50%, CG | 9.000 |
| | Potassium Nitrate, USP | 0.400 |
| **Blending Note:** add potassium nitrate into HP solution, mix until it is completely dissolved. | | |
| Phase II | Glycerine, USP | 7.750 |
| | Dehydrated alcohol, USP | 60.000 |
| | Eudragit L100-55 | 20.000 |
| | Nat. Peppermint Oil PE-5523 | 0.650 |
| | Eugenol, USP | 0.400 |
| **Blending Note:** Pre-prepare a solution with all ingredients from Phase II. Then mix the solution from Phase I with Phase II into one homogenous, viscous solution using a colloidal mill and vacuum. | | |
| Phase III | 40% Potassium Hydroxide, ACS | 1.800 |
| | 40% Potassium Hydroxide, ACS | Q.S to pH 6.5-7.0 |
| **Blending Note:** slowly add 40% (w/w) potassium hydroxide solution into the mixture of Phase I & II, mix until it becomes completely homogenous. Then use 40% Potassium Hydroxide to Q.S the final compound to pH 6.5-7.0 | | |

In this first example formulation, the viscosity of the final tooth whitening composition is greater than or equal to about 1000 cP measured at around 20 degree Celsius and the pH of the composition is within a range of about 6.5 to about 7.0.

### B. Non-Alcohol Based Anhydrous, Near Neutral pH, Peroxide Compound with Sensitivity Relief

Compared to the composition described above in section A, the non-alcohol based formulation described in this section replaces the alcohol ingredient with a non-alcohol, non-toxic, hydrophobic carrier such as petrolatum, polyethylene glycol (PEG), dimethyl silicone fluids, and the like. The formulation can be done in a double planetary mixer or Ross turbo-emulsifier that can handle high viscosity compounding under vacuum conditions. The vacuum condition may be applied partially during the process or may be applied during the entire formulation process. In some embodiments, a non-toxic, water-in-oil surfactant such as sorbitan monopalmitate may be added to the formulation when a high percentage of hydrophobic non-alcohol ingredient(s) is used.

An example formulation of a tooth whitening composition in the form of a non-alcohol based anhydrous, near neutral pH, peroxide composition with 4.5% (wt/wt.) hydrogen peroxide with sensitivity relief for teeth whitening is set forth in Table 2.

**TABLE 2**

| | **Ingredient** | **% w/w in part (a)** |
|---|---|---|
| Phase I | Hydrogen Peroxide, 50%, CG | 9.000 |
| | Potassium Nitrate, USP | 0.400 |
| | 10% Potassium Hydroxide, ACS | 0.023 |
| **Blending Note:** add potassium nitrate into HP solution, mix until it is completely dissolved. Then slowly add 10% (w/w) potassium hydroxide solution and mix fast until it becomes a completely homogenous solution. | | |
| Phase II | Sorbitan Monopalmitate, USP | 29.000 |
| | Petrolatum, USP | 60.527 |
| | Nat. Peppermint Oil PE-5523 | 0.650 |
| | Eugenol, USP | 0.400 |
| **Blending Note:** Pre-prepare an emulsion with all ingredients from Phase II using a Double Planetary Mixer. Then mix the solution from Phase I with Phase II into one homogenous, viscous emulsion in the Double Planetary Mixer under vacuum | | |

In this second example formulation, the viscosity of the final tooth whitening composition is greater than or equal to about 10,000 cP and the pH of the composition is within a range of about 6.5 to about 7.0.

### C. Mixture of Alcohol and Non-Alcohol Based Anhydrous, Near Neutral pH, Peroxide Compound with Sensitivity Relief

Compared to the first and second example formulations described above, this third example formulation uses a mixture of alcohol and non-alcohol (non-toxic, hydrophobic) ingredients as the carrier for the peroxide component. In some embodiments, compatible thickening agents may be added.

An example formulation of a tooth whitening composition in the form of mixture of alcohol and non-alcohol based anhydrous, near neutral pH, peroxide composition with 4.5% (wt/wt.) hydrogen peroxide with sensitivity relief is set forth in Table 3.

**TABLE 3**

| | **Ingredient** | **% w/w in part (a)** |
|---|---|---|
| Phase I | Hydrogen Peroxide, 50%, CG | 9.000 |
| | Potassium Nitrate, USP | 0.400 |
| | 10% Potassium Hydroxide, ACS | 0.023 |
| **Blending Note:** add potassium nitrate into HP solution, mix until it is completely dissolved. Then slowly add 10% (w/w) potassium hydroxide solution and mix fast until it becomes a completely homogenous solution. | | |
| Phase II | Sorbitan Monopalmitate, USP | 30.00 |
| | Petrolatum, USP | 53.00 |
| | Dehydrated alcohol, USP | 6.527 |
| | Nat. Peppermint Oil PE-5523 | 0.650 |
| | Eugenol, USP | 0.400 |
| **Blending Note:** Pre-prepare an emulsion with all ingredients from Phase II using a Double Planetary Mixer. Then mix the solution from Phase I with Phase II into one homogenous, viscous emulsion in the Double Planetary Mixer under vacuum. | | |

In this third example formulation, the viscosity of the final tooth whitening composition is greater than or equal to about 3,000 cP and the pH of the composition is within a range of about 6.5 to about 7.0.

The dispensing devices for the second and third formulations described above can be similar to those described for the first formulation, including a pen-style applicator, a single barrel/chamber syringe, a bottle, or any dispensing device that can dispense highly viscous fluids.

In some embodiments, the tooth whitening composition be applied directly to the teeth. In other embodiments, the tooth whitening composition can be combined with a delivery carrier, such as a strip of material, a dental tray, and/or a sponge material, and thereafter applied to the teeth.

In some embodiments, the tooth whitening compositions, such as those described above in sections A, B, or C, are allowed to remain in contact with the tooth for a period of time sufficient to whiten a tooth. A user may allow the tooth whitening composition to remain in contact with the tooth for a predetermined time by refraining from brushing the tooth, eating, drinking or otherwise wiping the whitening material from the tooth. The waiting period sufficient to whiten a tooth will depend on variables such as the whitening composition used, the extent and type of staining of the user's tooth and the amount of whitening composition used. For example, in some embodiments using any of the compositions described above, a wait time of about 10 to about 30 minutes may be recommended to achieve the desired tooth whitening result. In some embodiments, a tooth whitening composition may need to be applied once or twice a day and may continue to be applied for 14 treatments.

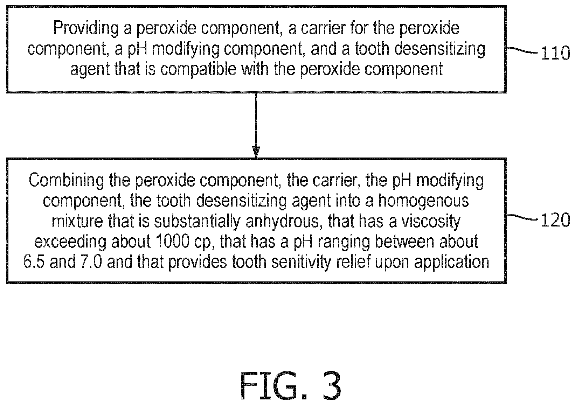

This disclosure is also directed to a method of manufacturing a composition for tooth whitening. FIG. 3 is a high-level flow chart of an exemplary method of manufacturing a composition for tooth whitening. At step 110, components of the tooth whitening composition are provided, including a peroxide component, a carrier for the peroxide component, a pH modifying component, and a tooth desensitizing agent that is compatible with the peroxide component.

At step 120, the components are combined into a homogenous mixture that is substantially anhydrous, that has a viscosity exceeding about 1000 cP, that has a pH ranging between about 6.5 and 7.0, and that provides tooth sensitivity relief upon application. During step 120, special mechanical compounding procedures and devices like a colloid mill, a double planetary mixer, or a Ross turbo-emulsifier, and a vacuum degassing, can be used to produce a viscous and homogenous finished product.

The inventive subject matter disclosed herein can be used for tooth whitening pens or other single cartridge-based tooth whitening products in oral healthcare. In some embodiments, the tooth whitening composition can be designed into a new form of anhydrous whitening compound with sensitivity relief for tray based whitening products, with light or without light activation. In some embodiments, peroxide percentages can be modified for producing chairside, take-home, or retail type peroxide based whitening products. In further embodiments, the formulation can be expanded to peroxide based sanitization applications by removing the desensitizing ingredient(s) from the formulation composition which cover household, industrial, institutional, health care and other areas.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one." In addition, singular references do not exclude a plurality. Thus, references to "a", "an", "first", "second" etc. do not preclude a plurality.

Reference signs in the claims are provided merely as a clarifying example shall not be construed as limiting the scope of the claims in any way.

The phrase "and/or," as used herein in the specification and in the claims, should be understood to mean "either or both" of the elements so conjoined, *i.e.,* elements that are conjunctively present in some cases and disjunctively present in other cases. Multiple elements listed with "and/or" should be construed in the same fashion, *i.e.,* "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, *i.e.*, the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e.,* to mean including but not limited to.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A composition for tooth whitening, comprising:
a peroxide component;
a carrier for the peroxide component;
a pH modifying component; and
a tooth desensitizing agent that is compatible with the peroxide component;
wherein the composition is a homogenous mixture that is substantially anhydrous, that has a viscosity exceeding about 1000 cP, that has a pH ranging between about 6.5 and about 7.0, and that provides tooth sensitivity relief to a user upon application.

2. The composition of claim 1, wherein a hydrophobic content of the homogenous mixture is more than 50% (w/w).

3. The composition of claim 1, wherein the peroxide component is selected from hydrogen peroxide, peracetic acid, or other suitable peroxide that is stable in the composition.

4. The composition of claim 1, wherein the peroxide component is hydrogen peroxide at a concentration of less than about 30% (w/w).

5. The composition of claim 4, wherein the concentration of hydrogen peroxide is in a range of about 2.0% (w/w) to about 14% (w/w).

6. The composition of claim 1, wherein the carrier for the peroxide component is ethanol.

7. The composition of claim 1, further comprising a thickening agent.

8. The composition of claim 7, wherein the thickening agent is selected from methacrylic acid copolymer, ammonia methacrylate copolymer, and polyvinyl acetate.

9. The composition of claim 1, wherein the carrier for the peroxide component is a non-alcohol, non-toxic, hydrophobic carrier.

10. The composition of claim 9, wherein the carrier for the peroxide component is selected from petrolatum, polyethylene glycol, and dimethyl silicone fluids.

11. The composition of claim 1, wherein the carrier for the peroxide component is a mixture of an alcohol carrier and a non-alcohol, non-toxic, hydrophobic carrier with a compatible thickening agent.

12. The composition of claim 1, wherein the tooth desensitizing agent is an inorganic salt.

13. A tooth whitening kit, comprising:
a composition for tooth whitening as claimed in claim 1; and
a dispensing device configured to receive the composition as a single part and adapted to dispense the composition onto a user's teeth.

14. A method for whitening teeth, comprising:
providing a composition for tooth whitening as claimed in claim 1; and
applying the composition to a tooth of a user for a desired amount of time.

15. A method of manufacturing a composition for tooth whitening, comprising the steps of:
providing a peroxide component, a carrier for the peroxide component, a pH modifying component, and a tooth desensitizing agent that is compatible with the peroxide component; and
combining the peroxide component, the carrier, the pH modifying component, the tooth desensitizing agent into a homogenous mixture that is substantially anhydrous, that has a viscosity exceeding about 1000 cP, that has a pH ranging between about 6.5 and 7.0, and that provides tooth sensitivity relief to a user upon application.
